# EUROPEAN PATENT SPECIFICATION

(11) **EP 3 516 381 B1**
(45) Date of publication and mention of the grant of the patent: **01.07.2020**
(21) Application number: 16775066.0
(22) Date of filing: 21.09.2016
(51) Int. Cl.: G01N 27/12

(54) **RESISTIVE METAL OXIDE GAS SENSOR COATED WITH A FLUOROPOLYMER FILTER**
RESISTIVER METALLOXIDSENSOR ÜBERZOGEN MIT EINEM FLOROPOLYMERFILTER
CAPTEUR RESISTIF A OXIDE DE METAL AVEC UNE COUCHE FILTRE EN FLUOROPOLYMERE

(43) Date of publication of application: 31.07.2019
(73) Proprietor: Sensirion AG, 8712 Stäfa (CH)
(72) Inventor: ANDERSSON, Pernilla, 8712 Stäfa (CH); ROTZETTER, Aline, 8712 Stäfa (CH)
(74) Representative: Ragot, Sébastien Pierre
(86) International application number: PCT/CH2016/000122
(87) International publication number: WO 2018/053655

(56) References cited:
- EP-A1- 2 778 667
- US-A1- 2008 302 672
- US-A1- 2016 025 517
- Sgx ET AL: "SGX Metal Oxide Gas Sensors (How to Use Them and How They Perform) INTRODUCTION", , 14 July 2014 (2014-07-14), pages 1-5, XP055370292, Retrieved from the Internet: URL:https://www.sgxsensortech.com/content/ uploads/2014/08/AN-0172-SGX-Metal-Oxide-Ga s-Sensors-V1.pdf [retrieved on 2017-05-08]
- S C GADKARI ET AL: "Solid State Sensors for Toxic Gases Solid State Sensors for Toxic Gases", NATIONAL CONFERENCE ON SENSORS TECHNOLOGY (NCST-2004), 28 December 2004 (2004-12-28), pages 49-60, XP055369947,

## Description

### BACKGROUND

The invention relates in general to the field of resistive metal oxide gas sensors and methods of operation thereof. In particular, it concerns a resistive metal oxide gas sensor covered with a filter layer.

Gas sensors are known for detecting gases based on a variety of techniques. Such sensors include at least one sensing (or active) element sensitive to the presence or concentration of one or more gases. One known class of gas sensors are catalytic gas sensors, or pellistors, which detect the presence of combustible gases. In pellistors, a detector element provides an electrical resistance measurement dependent on the presence of a combustible gas. In particular, the resistance in the detector element varies with changes in temperature produced by the catalytic oxidation of a combustible gas if present. To facilitate the combustion of the gas, the sensors are operated at an elevated temperature, i.e., typically larger than 300°C, for example from 450 °C to 750°C.

Another class of gas sensors involves chemiresistors, i.e., materials for which the electrical resistance changes in response to changes in their direct chemical environment. Chemiresistors are sometimes defined as relying on direct chemical interactions between the sensing material and the analyte. More general definitions of chemiresistors, however, include materials for which the electrical resistance changes in response to any type of interactions (chemical, hydrogen bonds, van der Waals, etc.) in their direct environment.

In each case, the sensing material may contain a metal oxide material, which may for instance include one or more of tin oxide, zinc oxide, titanium oxide, tungsten oxide, indium oxide and gallium oxide.

Metal oxides may be used for the detection of analytes such as volatile organic compounds (VOCs), carbon monoxide, nitrogen dioxide, methane, ammonia or hydrogen sulphide.

In metal oxide sensors, gaseous analytes interact with the (heated) metal oxide layer. As a result of the interaction, the conductivity of the sensitive film may change, and the change may be measured. Such gas sensors are also referred to as "high temperature chemiresistors" for the reason that a chemical property of the analyte is converted into an electrical resistance at high temperatures of the sensitive film.

Such sensor devices require a heater to heat the sensing material. They can be integrated onto a semiconductor substrate, in which case the heater is advantageously arranged on a membrane over an opening in the semiconductor substrate, thereby reducing the thermal loss as compared to devices where the heater is arranged over the bulk of the substrate material. Arranging the heater on a membrane has several advantages, such as reducing power consumption, increasing sensitivity and reducing the time required for switching on the device.

WO2014012951 (A1) describes a gas sensor comprising an active element and a filtering cap, wherein the cap comprises a porous filtering element for counteracting the ingress of one or more atmospheric gases to the active element. There is a separation between the active element and the filtering element of the cap. In addition, the filtering element comprises a mechanically densified powder of filtering material. Namely, the filtering cap comprises a support formed of a suitably rigid material which defines a socket for the filtering element. Together the support and filtering element of the filtering cap define a cavity or gap in which the active element is disposed together with an associated hotplate and circuitry.

The separation, or gap, between the filtering element and the active (or sensing) element has been found advantageous to avoid thermal losses from the active element. Thermal losses may be of concern in the design of small-scale active elements, for example based on MEMS technology. In addition, and as it may be realized, such a separation may be necessary to avoid damages to the filtering cap. Thanks to the separation, or gap, the sensing element can be operated at elevated temperatures, e.g., higher than 350C or even 400C, without damaging the filtering cap.

A support of a filter separated from the sensing element requires a certain rigidity and thickness, which together with the separation gap results a height of the device that may be disadvantageous for small consumer electronic devices.

### SUMMARY

According to a first aspect, the present invention is embodied as a resistive metal oxide gas sensor. The sensor notably comprises a support structure and a patch of sensing material arranged on the support structure or partly housed therein. The patch comprises a metal oxide material. Electrodes are in electrical communication with the patch. The sensor further comprises a heater, in thermal communication with the patch, and a selective gas-permeable filter. The selective gas-permeable filter comprises a fluoropolymer. A first part of an external surface of the patch covers a part of the support structure, while a remaining part of said external surface is coated by the selective gas-permeable filter, so as to form a coated patch of sensing material.

Since the selective gas-permeable filter coats the remaining part of the external surface of the patch of sensing material, there is no separation, no gap, i.e., no dead volume between the filter and the sensing material. Such a structure challenges the commonly accepted approach, according to which a filter should be separated from the heated sensing material, especially when the filter contains a thermally unstable material. The present design eases the fabrication process, while it allows fast response times by the sensor. Meanwhile, present inventors have realized that the above sensor could be safely, yet efficiently operated already at moderate temperatures, e.g., at less than 300C, to prevent damages to the fluoropolymer material used in the filter.

In particular, the gas sensor may, in embodiments, further comprises a temperature controller. The latter is an electronic circuit or processing unit connected to the heater, and is programmed, designed, adapted or configured to prevent the heater to heat the coated patch to a temperature exceeding 300C.

In more sophisticated embodiments, the gas sensor further comprises a temperature sensor arranged in the resistive metal oxide gas sensor for estimating a temperature of the coated patch. The temperature sensor may form part of the heater. A temperature controller is connected to the heater and, if necessary, to the temperature sensor. The temperature controller may form, together with the heater and, if necessary, the temperature sensor, a feedback loop, so as to maintain a temperature of the coated patch at a substantially constant value, below a glass transition temperature of the fluoropolymer, in operation.

In preferred embodiments, the feedback loop maintains, in operation, the temperature of the coated patch at a substantially constant value between 100C and 240C, and preferably between 150C and 200C.

Preferably, the fluoropolymer comprises Teflon, which provides satisfactory filtering properties. For example, amorphous fluoroplastics (Teflon AF), e.g., Teflon AF 1600 or Teflon AF 2400 can be used.

In embodiments, the filter comprises one or more layers of materials, including a fluoropolymer layer (the latter comprising said fluoropolymer). An average thickness of the fluoropolymer layer is between 10nm and 50µm, and preferably between 10nm and 2µm.

Preferably, the average thickness of the sensing material of the patch is between 0.1µm and 50µm, and more preferably between 0.5µm and 5µm.

The small thicknesses of the fluoropolymer layer and/or the sensing material allow, together with the absence of separation, the height of the sensor to be reduced, which in turn eases its integration into larger devices (e.g., CMOS circuitry, for integration into home automation devices, consumer electronics, smartphones or other mobile devices). Shallow sensors are furthermore beneficial for batch manufacturing.

As present inventors have further realized, using a fluoropolymer layer, whose thickness is larger than 300nm, will substantially prevent reactive gases such as O₃ or NO₂ to reach the sensing material. Thus, in applications, the average thickness of the fluoropolymer layer is between 300nm and 50µm, to block ingress of such reactive gases. On the contrary, a thickness between 10nm and 300nm may be sought to specifically sense such gases.

In particular applications, the average thickness of the fluoropolymer layer is between 50nm and 250nm, and the metal oxide material comprises SnO₂, doped with 0.01 - 1.0 Wt% platinum and/or palladium. Such a solution has been found particularly efficient for sensing ozone.

In embodiments, the metal oxide gas sensor further comprises an evaluation unit, e.g., directly integrated onto or into said support structure. The evaluation unit is an electronic circuit or processing unit connected to the electrodes to receive signals therefrom. It is programmed, designed, adapted or configured to determine values indicative of an electrical conductivity of the metal oxide material based on signals received from the electrodes, so as to identify the gases sensed.

In embodiments, the selective gas-permeable filter comprises a fluoropolymer layer and an interlayer. Namely, the remaining part of the external surface of the patch is coated by the interlayer, and the latter is coated by the layer of fluoropolymer. The interlayer may for instance be catalytically inactive or, at least less active than the fluoropolymer, to help preventing a premature decomposition of the fluoropolymer. The interlayer typically comprises a chemically inert material, e.g., an insulator such as SiO₂. In variants, it may comprise a polymer.

Preferably, the first part of the external surface coats said part of the support structure, e.g., it extends flat on an exposed surface thereof, as in a "membrane" configuration, which has several advantages, such as reducing power consumption, increasing sensitivity and reducing the time for switching on the device.

The metal oxide gas sensor can also be embodied as a "multipixel" resistive metal oxide gas sensor, to concomitantly sense several types of gas molecules. Namely, such a sensor comprises one or more support structures (including the support structure evoked earlier) and a set of patches of sensing material (these including said patch), wherein each of the patches comprises a metal oxide material and is arranged on or partly housed in one of the support structures. In addition, a set of electrodes is provided (including said electrodes), whereby each of the patches is in electrical communication with a subset of the electrodes of the set of electrodes. One or more heaters (including said heater) are in thermal communication with the patches of sensing material. Finally, this sensor comprises one or more selective gas-permeable filters (including the selective gas-permeable filter mentioned above), which comprise, each, a fluoropolymer. Consistently with the sensors discussed earlier, a first part of the external surface of each of the patches covers a part of a support structure, while a remaining part is coated by a selective gas-permeable filter, so as to form distinct, coated patches of sensing material. The coated patches differ in terms of dimensions and/or compositions of their respective filters and/or respective metal oxide materials, or the configuration of the electrodes may also differ, from one patch to the other, so as to be able to sense different types of gases.

Preferably, such a sensor comprises several, distinct selective gas-permeable filters, which coats two or more of the patches. This way, distinct, coated patches of sensing material are obtained, whose respective filters differ in terms of dimension and/or composition, while the sensing materials used for the patches or their respective electrodes need not necessarily differ, which eases both the fabrication and the operation processes (notably in terms of heating the sensing material) while still enabling selectivity.

The present invention may for instance be embodied as an electronic device, in particular a home automation device, a consumer electronics device, a mobile phone, a tablet computer or a watch, comprising any resistive metal oxide gas sensor such as discussed above.

According to another aspect, the invention is embodied as method of operating a resistive metal oxide gas sensor according to any of the above embodiments. Basically, such a method revolves around heating a coated patch of sensing material and determining values indicative of an electrical conductivity of the sensing material of the patch, based on signals received from the electrodes and, this, while heating the coated patch.

In embodiments, the patch is heated to a temperature that is between 100C and 300C, to ensure a reasonable reactivity of the metal oxide material, while preventing damages to the filter.

Preferably though, this temperature is maintained at a desired value, below a glass transition temperature of the fluoropolymer, e.g., between 100C and 240C, or between 150C and 200C.

In preferred embodiments, a "multipixel" sensor is used, such that signals pertaining to distinct types of gas molecules can be acquired, as filtered by one or more selective gas-permeable filters and sensed via distinct, coated patches of sensing material.

Devices, apparatuses and methods embodying the present invention will now be described, by way of non-limiting examples, and in reference to the accompanying drawings.

### BRIEF DESCRIPTION OF SEVERAL VIEWS OF THE DRAWINGS

FIG. 1A is a 2D cross-sectional view of a gas sensor in a membrane configuration, according to embodiments;
FIG. 1B is a top view of the device of FIG. 1A;
FIG. 1C is a 3D view of this device;
FIG. 1D is another 2D cross-sectional view of this device, which focuses on the later structure of the coated patch;
FIG. 2 is a top view of a gas sensor that comprises multiple patches, each in a bridge configuration, according to other embodiments;
FIGS. 3A and 3B are 3D views of a smartphone that includes a gas sensor and an evaluation unit to determine values indicative of the electrical conductivity of the patch of sensing material, according to embodiments;
FIG. 4 shows a smartwatch equipped with a gas sensor, according to other embodiments;
FIG. 5 is a 2D cross-sectional view depicting a gas sensor, lodged in a cavity of a mobile device such as depicted in FIG. 3 or 4, according to embodiments;
FIG. 6 is a plot representing measured resistances of different patches of sensing materials (coated with a fluoropolymer, or not), as measured after exposure to siloxane, and while sensing different types of gas molecules (i.e., dihydrogen and ethanol);
FIG. 7 is a plot representing the resistances of patches coated with fluoropolymer layers of various thicknesses, while sensing ozone;
FIGS. 8 and 9 illustrate the influence of temperature on the measured resistances of coated and uncoated patches when sensing different types of gas molecules (i.e., dihydrogen and ethanol); and
FIG. 10 is a flowchart illustrating high-level steps of a method of operating a gas sensor device, according to embodiments;

The accompanying drawings show simplified representations of devices or parts thereof, as involved in embodiments. Technical features depicted in the drawings are not necessarily to scale. Similar or functionally similar elements in the figures have been allocated the same numeral references, unless otherwise indicated.

### DETAILED DESCRIPTION OF EMBODIMENTS OF THE INVENTION

The following description is structured as follows. First, general embodiments and high-level variants are described (sect. 1). The next section addresses more specific embodiments and technical implementation details (sect. 2).

### 1. General embodiments and high-level variants

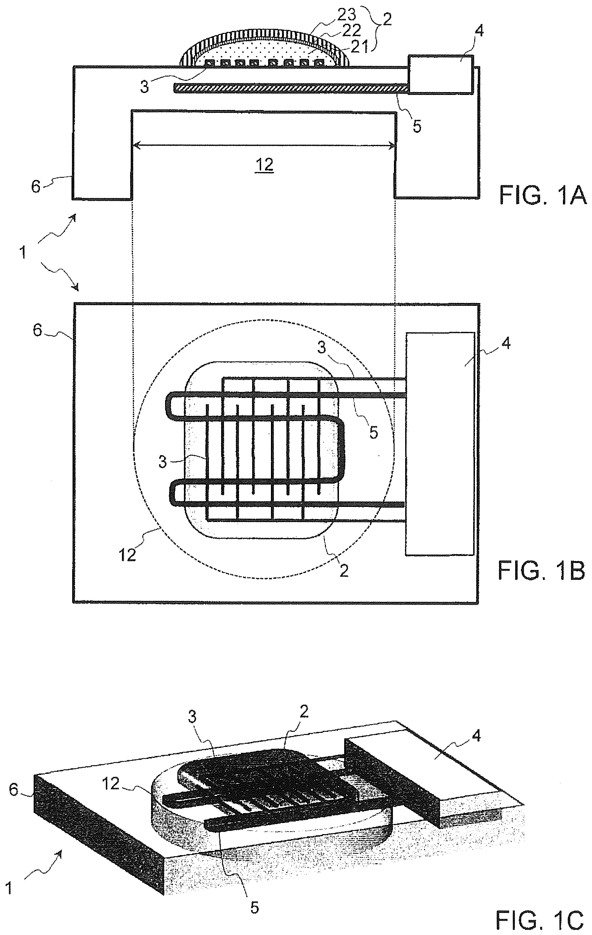

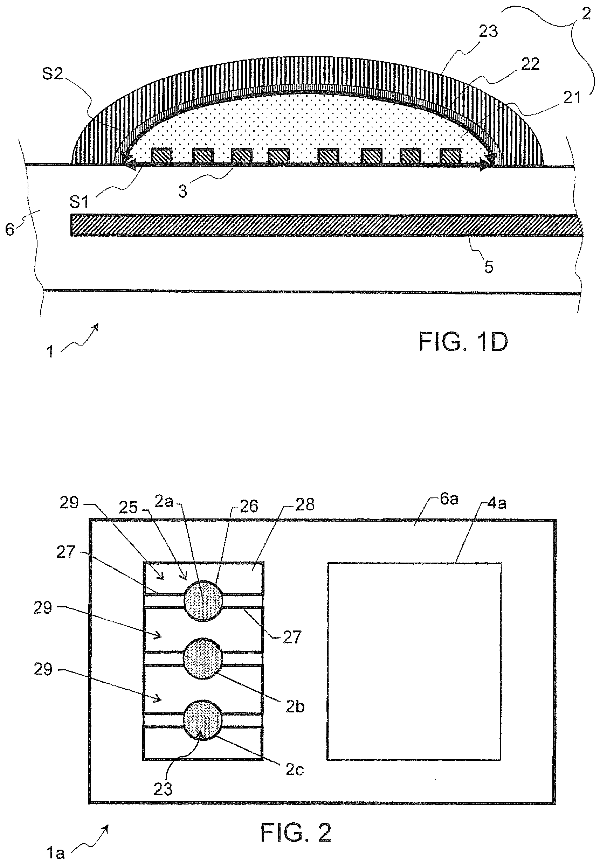

In reference to FIGS. 1, 2 and 5, an aspect of the invention is first described, which concerns a resistive metal oxide gas sensor 1,1a.

The gas sensor comprises a support structure 6, 6a, 27, 29, which typically includes a structured substrate, e.g., of silicon. The support structure 6 may have a membrane configuration, as in FIG. 1. In variants such as illustrated in FIG. 2, the support structure 6a has a bridge configuration 27, 29. Other configurations of the support structure may be contemplated.

The gas sensor further comprises a patch 21 of sensing material. The latter comprises a metal oxide material, or MOX, 21. The patch 21 may be arranged on the support structure 6, 6a, 27, 29. It may for instance extend on an exposed surface of the support structure 6, e.g., overlaid flat on an upper surface thereof, as in FIG. 1, or extend on substructures thereof, as in FIG. 2. In other variants, the patch may be partly housed in a cavity of the substrate. In all cases, a residual portion S2 of the (external) surface of the oxide material is not concealed by or in the support structure, and is oriented so as to enable sensing by the oxide material.

The patch 21 can be regarded as a small piece of MOX material, e.g., a layer portion 21 deposited or patterned onto the support structure 6, 6a, 27, 29. The patch 21 is typically a flattened object, to ease integration of the sensor. The patch 21 may for instance have a disk, ovoid or, still, a rectangular shape, or more generally be an essentially convex object, to ease coating thereof by the filter 22, 23.

Beyond the examples cited in the background section, preferred MOX materials 21 comprise SnO₂, ZnO and/or WO₃, and preferably comprises dopants too, the latter comprising one or more of Pd, Pt, Rh, Ir, Re, V, Ni, Au, and Co.

Electrodes 3 are arranged in the sensor, so as to be in electrical communication with the patch 21 of sensing material. They may be formed out of a platinum or gold layer, which metals are well suited for forming stable electrodes. Electrodes may for instance be in an interdigitated configuration, as illustrated in FIG. 1, for reasons that will become apparent later. Thus, the patch 21 may advantageously have shape (e.g., convex) that spans a region that covers or includes interdigitated fingers of the electrodes 3. The latter may notably be covered by the patch (if partly integrated into the substrate 1) or integrated therein (if patterned onto the substrate 1), to ensure proper electrical communication therewith. In this respect, the electrodes may be considered as part of the support structure a part of which is covered by the patch.

A heater 5 is in thermal communication with the patch 21, to operate the sensing material at a required temperature. A heater 5 is a resistive heating element. For example, one may use a heater of tungsten, i.e., a heater comprising at least 50%, in particular at least 90%, of tungsten, to best withstand high temperatures. Several heaters may be provided, to heat a plate (e.g., a membrane, or a bridge), on which the patch 21 is arranged. In variants, the heater may be embodied as a hotplate, which is resistively heated, without additional resistive elements being needed. The heater can be used to heat the patch and, if necessary, to furthermore control the temperature of the patch 21. Thus, no additional temperature sensors need necessarily be provided.

In addition, the gas sensor includes a selective gas-permeable filter 22, 23 (also referred to herein as a "gas-permeable filter", or simply "filter"), which comprises a fluoropolymer 23. As seen in FIG. 1A or 1D, the gas-permeable filter 22, 23 may have a multilayered structure, e.g., it may include a thin interlayer 22 between the fluoropolymer layer 23 and the sensing material 21 of the patch. In variants, the filter is made of a single fluoropolymer layer 23 that directly coats the MOX 21 (i.e., no interlayer is provided).

Irrespective from any specific embodiments, the filter granting or denying access to the cavity and hence to the sensing element is a size selective filter. Hence, the filter performs a separation between gas molecules allowed to pass the filter and gas molecules blocked from passing through the filter subject to the size of the gas molecules. The filter preferably is made from a material that is inert, i.e. a material that is not or not substantially reactive. Therefore, gas molecules - irrespective if passing through the filter or not - do not react with the filter material but are blocked because of their size. This has the advantage that the filter material does not degrade over time compared to a filter material chemically reacting with non-desired gas molecules.

Hence, the selection between gases to pass the filter and gases blocked by the filter is made according to the size of the respective gas molecules. It is preferred that the size selective filter filters subject to the molecule size of gas encountering the size selective filter. Preferably, the size selective effect of the filter is determined by a size of pores in the filter material. In particular, the size of the pores in the filter material is dimensioned dependent on a size of a molecule of the gas to be detected and hence desired to pass the filter. In particular, the size of a majority of the pores in the filter material is dimensioned to let a / the molecule/s of the gas to be detected pass and is dimensioned to block a / the molecule/s of one or more other gases, that are to be prevented from entering the cavity. Preferably, the size of a majority of the pores in the filter material is dimensioned to exceed the size of a molecule of the gas to be detected, and is dimensioned smaller than the size of a molecule of a gas to be blocked from passing the filter. Preferably, the material of the filter is selected and / or designed to such filtering effect.

Hence, it is preferred that the filter is permeable for the gas to be detected by the sensing element and non-permeable for one or more other gases. Such other gases may in particular include siloxane and variants thereof which are prone to react with the material of the sensing element and degrade its sensing capabilities over time. Hence, the size selective filter counteracts ingress of one or more types of atmospheric gases to the sensing element, i.e. preferably, the MOX, while allowing other types of gas molecules to diffuse there through and reach the sensing element. Thus, the filter makes it possible to counteract the ingress of inhibiting and/or poisoning species to the sensing element, and this operates by size exclusion.

In a very preferred embodiment, the size of a majority of the pores in the filter material is 1 nm or less. This dimension, in particular, is preferred in case the sensing element is configured to sense one or more of CO, Ethanol, H₂, H₂S. Here, gas molecules of the subject gases of interest are sufficiently small to pass the filter while many kinds of siloxane molecules are too big in size to pass, and hence, will be blocked by the filter.

Whenever it comes to the dimensioning or pore sizes, it is understood, that preferably all pores of the subject material fulfil the dimensional requirements. However, owed to manufacturing, not all but a lower percentage of pores may only fulfil the dimensional requirements, preferably more than 99%. In a worst case, it is preferred that a majority of the pores in the filter material fulfil the dimensional requirement in order to achieve at least a better selection than with conventional approaches.

By "selective gas-permeable filter", it is meant a filter that is designed to counteract ingress of one or more types of atmospheric gases to the sensing element, i.e., the MOX, while allowing other types of gas molecules to diffuse therethrough and reach the sensing element 21. Thus, the filter makes it possible to counteract the ingress of inhibiting and/or poisoning species to the sensing element. All the more, the filter 22, 23 lowers the background and therefore allows better SNRs to be eventually be obtained.

A fluoropolymer is a fluorocarbon-based polymer that exhibits multiple carbon-fluorine bonds. It usually has a high resistance to solvents, acids, and bases, so that it can advantageously be used for the present purpose. Present fluoropolymers may notably include one or more amorphous fluoroplastics, such as the so-called AF amorphous fluoroplastics from Chemours™. Preferred is an amorphous fluorinated polymer that has a relatively high glass transition temperature (Tg), i.e., of more than 100C, for example of about 160C or 240C, such as the so-called AF 1600 and AF 2400 from Chemours™.

For the filter, preferably an amorphous Teflon AF alike material is used according to row no. 4 of the following Table I showing preferred compositions for the filter material in each row, Row no. 4 thereby denotes an umbrella term, the other individual material compositions according to preferred embodiments in row no. 1 to row no. 3 can be subsumed under. Hence, the filter fluoropolymer may in one embodiment be a homopolymer, see row no. 3, while in the other embodiments, it is a copolymer, see row no. 1 and row no. 2.

**Table I**

| No | Name | Component 1 | Component 2 |
|---|---|---|---|
| 1 | Teflon AF 2400 | 2,2-bistrifluoromethyl-4,5-difluoro-1,3-dioxole | tetrafluoroethylene |
| 2 | Teflon AF 1600 | 2,2-bistrifluoromethyl-4,5-difluoro-1,3-dioxole | tetrafluoroethylene |
| 3 | PDD homopolymer | 2,2-bistrifluoromethyl-4,5-difluoro-1,3-dioxole | n/a |
| 4 | **Teflon AF alike** | **2,2-bistrifluoromethyl-4,5-difluoro-1,3-dioxole** | **tetrafluoroethylene** |

In the following Table II properties for the individual material compositions of above row no. 1 to 4 are illustrated, wherein
- Comp 1, mol% denotes the mole fraction of the Component 1;
- Comp 2, mol% denotes the mole fraction of the Component 2;
- FFV denotes the free fraction per volume;
- Tg °C denotes the glass transition temperature of the resulting material, in °Celsius; and
- Tmax denotes a maximal temperature at which polymers do not show notable degradation (i.e. thermally stable), in °Celsius.

**Table II**

| No | Name | Comp 1, mol % | Comp 2, mol % | FFV, % | Tg, °C | Tmax, °C |
|---|---|---|---|---|---|---|
| 1 | Teflon AF 2400 | 87 | 13 | 33 | 240 | 360 |
| 2 | Teflon AF 1600 | 65 | 35 | 30 | 160 | 360 |
| 3 | PDD homopolymer | 100 | 0 | >33 | 335 | 360 |
| **4** | **Teflon AF alike** | **100-20** | **0-80** | | **80-250** | 360 |

As better seen in FIG. 1D, a first part S1 (e.g., the basis surface) of the external surface of the patch 21 covers a part of the support structure 6, including the electrodes 3. Yet, this first part S1 preferably coats said part of the support structure. This makes sure there is no dead volume on the support structure side either. All the more, such a configuration is usually simpler to fabricate, as a mere film deposition is needed here, which is simpler to achieve than a membrane film, which requires support structures. For example, and as illustrated in FIGS. 1, the patch 21 may extend flat on an exposed surface of the support structure 6, which have several advantages, in terms of power consumption, sensitivity and time for switching on the device.

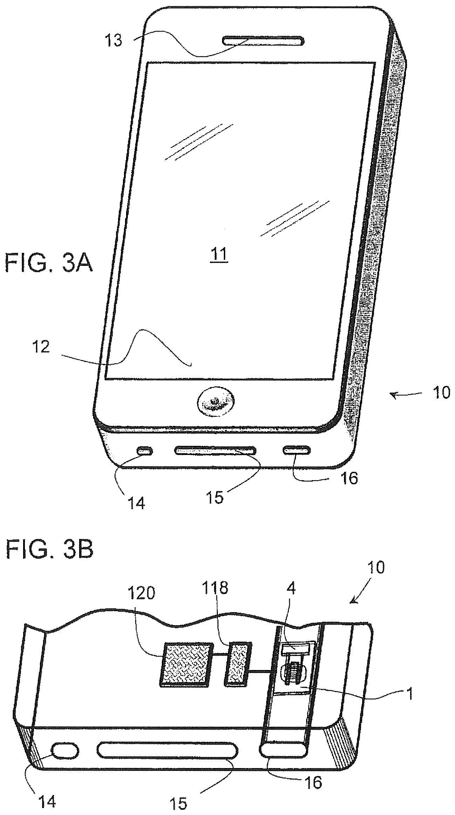

Moreover, the remaining part S2 of the external surface of the sensing material 21 is coated by the gas-permeable filter 22, 23, so as to form a coated patch of sensing material. For example, the sensor of FIG. 1 exhibits a single coated patch 2, whereas the bridge sensor of FIG. 3 shows three coated patches 2a - 2c.

Said "remaining part" S2 of the external surface of the patch 21 refers to any portion of the patch 21 that does otherwise not cover the lower structure 6, 6a, 27, 29, 3, i.e., any portion not exposed to the support structure. While it typically comprises only one such remaining part S2, as assumed in FIG. 1, said remaining part S2 may actually comprise two or more disjoint portions, depending on the exact configuration of the patch 21. The same considerations apply to the first part S1 of its external surface. This depends on the complexity and shape of the support structure, the shape of the patch 21, whether the latter is partly housed in a cavity of the substrate 1, etc.

Thus, more generally, the gas-permeable filter 22, 23 is coated on any remaining part S2 of the external surface of the patch 21, i.e., on any portion thereof that does otherwise not cover the structure 6, 6a, 27, 29, That is, the filter 22, 23 is coated on any remaining surface portion that is not exposed to the support structure 6, 6a, so as to enable sensing from the opposite side. The filter 22, 23 may consist of a single layer or multiple layers coated on said remaining part S2 of the surface, while the first part S1 is concealed, if not coated as well, by the support structure 6, 6a. As a result, the patch 21 is concealed in the object formed by the support structure 6, 6a (or by substructures 27, 29 thereof) and the filter 22, 23.

Note that the words "coating", "coats", "cover", "covering", etc., as used above, refer to structural aspects of the present devices, without prejudice as to the order of fabrication steps used to fabricate such devices. Yet, a patch 21 is normally formed after having formed the support structure, e.g., by depositing or patterning the patch 21 thereon.

In embodiments, the present sensor can be operated as a chemiresistor, wherein the MOX material 21 changes its electrical resistance in response to changes occurring in the filter 22, 23, which allows certain molecules to reach the MOX, leading to chemical interactions between the sensing material 21 and the analytes. In other embodiments, one may rely on a calorimetric determination of the analyte. In still other embodiments, the patch of sensing material can be used for two purposes, namely: (i) as a chemiresistor that changes its electrical conductivity in the presence of the analyte; and (ii) as a catalyst in a calorimetric determination of the analyte.

The above sensor preferably includes circuitry, integrated therewith, to heat the heater and perform resistive measurements, i.e., to measure an electrical conductivity and/or resistivity of the patch.

A plurality of patches (or "pixels") may be provided, with different filters, electrode designs and/or MOXs, to concomitantly sense several types of gas molecules, as in, e.g., FIG. 2. A subset of the pixels may be coated by a single filter layer extending over the whole subset, or by respective and distinct filter layers that possibly have different compositions, to selectively allow molecules to reach each sensing pixel, as discussed later in detail.

The fluorinated coating 22, 23 protects the MOX 21 against aggressive chemicals such as acids or bases, and further prevents, by design, buildup of solids and liquids on the MOX surface S2.

Remarkably, the structure of the present gas sensors 1, 1a, wherein the filter 22, 23 directly coats the sensing material 21, excludes any substantial air gap, or other separation with a thermal insulator, between the sensitive MOX patch layer 21 and the filter 22, 23, contrary to solutions adopted in the art, such as described in the background section. The absence of significant dead volume between the sensitive MOX layer 21 and the filter 22, 23 eases the fabrication process (a mere film deposition is needed), while it ensures fast response times of the sensor. Meanwhile, and as present inventors have further realized, the above sensor can nevertheless be safely, yet efficiently operated already at moderate temperatures, e.g., at less than 300C, which prevents damages to the fluoropolymer material 23, despite the lack of separation, or gap.

Fluoropolymers, such as Teflon, as used herein, may not be thermally as stable as other materials that are usually used in filters for MOX sensors. However, this issue is alleviated by the comparably low operation temperature of the sensor. The sensor can be continuously heated or, if necessary, it can be intermittently heated, by way of short heat pulses. The resulting, average temperature of the patch can nevertheless be maintained in a safe range, as needed to prevent damages to the filter. In all cases, the detection of target gases is already possible at relatively low temperatures e.g., less than 300C (or even less than 240C, or 160C, as discussed below), whereas known gas sensors are typically operated at higher temperatures.

As a surprisingly enough example, present inventors have observed that a MOX material directly coated with a fluoropolymer and operated at a maximal temperature of 200C provides better results in terms of sensing than the same MOX material, uncoated, and even when the latter is heated above 200C.

Referring now to FIG. 3B, in addition to FIGS. 1, 2 and 5, the gas sensor 1, 1a may, in embodiments, further comprise a temperature controller. The latter may notably be embodied as an CMOS circuit 4, 4a, integrated in the same support structure (substrate), or as an external component 118, 120, connected to components of the support structure 6, 6a, see for example FIG. 3B. The temperature controller is, however, preferably integrated directly in the support structure 6, 6a, to off-load processing from external components and allow faster readouts, as discussed later in detail.

In all cases, the temperature controller is an electronic circuit or a processing unit that is connected to the heater 5. The temperature controller is configured to prevent the heater 5 to heat the coated patch 2, 2a - 2c to a temperature that would damage the coating, e.g., a temperature exceeding 300C. The temperature controller may be programmable, or otherwise designed (e.g., hardcoded) to achieve the same.

Note that the above value of 300C may be subject to an accuracy of ± 25C (or ± 10C, depending on the method used), owing to difficulties in precisely estimating the temperature at the patch, notably because of heat losses between the heater and the patch. As inventors observed, a critical temperature above which the fluoropolymer layer starts decomposing or otherwise become unstable would actually be of about 360C. Thus, the upper temperature should preferably not exceed 360C. More preferably yet, one uses an upper limit of 300C, to favor the longevity of the sensor.

Assuming that all other parameters are known or determinable, the maximal power delivered by the temperature controller may be determined so as to prevent the temperature of the patch to exceed this upper temperature limit. In this case, there is no need of a feedback loop, or any other active temperature monitoring.

In more sophisticated variants, the temperature controller 4, 4a, 118, 120 may form a feedback loop controlling a current passed through the heater 5, in order to maintain a temperature at the location of the patch 21 at a desired value. Namely, the gas sensor 1, 1a may further comprise a temperature sensor 5 arranged in the resistive MOX gas sensor 1, 1a, so as for the temperature controller to be able to estimate a temperature of the coated patch 2, 2a - 2c. The temperature sensor may for example be used to measure a temperature at a location of said patch 21 on the support structure, from which the actual temperature of the coated patch 2, 2a - 2c may be extrapolated. This temperature sensor can be an individual component. In variants, and as evoked earlier, the temperature sensor may form part of another component, e.g., the heater 5. Indeed, it may also be incorporated into the heater 5 as the heater's resistance is also indicative of the temperature at the location of the patch. In all cases, the device may yield a signal that is indicative of an absolute temperature or of a relative temperature. Several temperature sensors may be used, if necessary.

Thus, the temperature controller 4, 4a, 118, 120 may be connected to the heater 5 and, if necessary, to the temperature sensor 5. Again, the temperature controller may be an electronic circuit or a processing unit. It is configured to form, together with the heater 5 (and, if necessary, a temperature sensor), a feedback loop, which, in operation, maintains a temperature of said the patch 2, 2a - 2c at a desired value, e.g., at a substantially constant value.

In addition, the gas sensor 1, 1a may, in embodiments, further comprise an evaluation unit 4, 4a, 118, 120. Again, the evaluation unit may notably be embodied as an integrated CMOS circuit 4, 4a, or as an external component 118, 120, see FIG. 3B. It is, however, preferably integrated directly in the support structure 6, 6a, for the same reasons as mentioned earlier in respect to the temperature sensor. The evaluation unit 4, 4a, 118, 120 can similarly be regarded as an electronic circuit or processing unit. It is connected to electrodes 3 of the sensor, so as to receive signals therefrom, and to accordingly determine values indicative of an electrical conductivity of the MOX material 21. In all case, the evaluation unit may be programmed (or be programmable), designed (e.g., in hardware), or otherwise adapted to determine such values, i.e., the evaluation unit is configured to determine values indicative of an electrical conductivity of the MOX, based on signals it receives from the electrodes.

The operation temperature of the sensor will in general be set in the interval [100C, 360C], or preferably in the interval [100C, 300C], as noted earlier. The upper and lower temperature limits depend on the MOX used and the thermal stability of the filter. They may again be subject to an accuracy that typically varies between ± 10C and ± 25C. In fact, and as it may be realized, the upper limit is determined by the thermal stability of the fluoropolymer used in the filter, which may typically start decomposing, e.g., by oxidation, at temperatures above 360C. Conversely, the lower limit is determined by the MOX material, which must sufficiently heated to be "active".

Thus, to cope with potential inaccuracies in the temperatures measured, each lower temperature limit as mentioned herein (e.g., 100C) may need be increased by, e.g., 25C, whereas preferred upper temperature limits mentioned herein (e.g., 360C, 300C, or 160C or 240C, see below) may need be lowered by the same amount, e.g., 25C, in practice, to make sure that the MOX be sufficiently active and prevent damages to the fluoropolymer. Note that, owing to the present geometry 21 - 23 of the filter 22, 23 with respect to the MOX material 21, the temperatures of the MOX material and the filter can be assumed to be the same, in operation.

Classes of MOX materials may allow lower operation temperatures, such that the above interval may, in embodiments, narrow down to [100C, 240C], or even [150C, 200C]. For continuous operation (at constant temperatures), one preferably maintains this temperature below the glass temperature (e.g., 240C for Teflon AF 2400 or 160C for AF 1600). Yet, the MOX may be intermittently heated, while its average temperature may be maintained in a desired range. In addition, the patches may occasionally need be heated for short periods to temperatures above the glass transition temperature, e.g., to clean the sensor, for example to burn VOC remains.

In embodiments, the fluoropolymer 23 may further comprises particles (filler) of a polymer, e.g., a fluoropolymer or of a catalytically active material (MOXs, manganese oxides, Pd, Pt, etc.). Using a filler can help to improve selectivity properties in respect of certain analytes.

More generally, the filter layers 22, 23 should exhibit good performances in terms of thermal, chemical, mechanical, and electrical stability. In embodiments, the gas-permeable filter 22, 23 comprises one or more layers of materials, the latter including a layer 23 of the fluoropolymer, as seen in FIG. 1D. The average thickness of the fluoropolymer layer 23 is preferably between 10nm and 50µm, which is sufficient for most applications. More preferably, it is between 10nm and 2µm, e.g., to lower the height of the device and ease integration. A thickness of 2µm still enables satisfactory selectivity properties in practice.

In addition, the average thickness of the sensing material of the patch is preferably between 0.1µm and 50µm, and more preferably between 0.5µm and 5µm. The thicknesses of the fluoropolymer layer and/or the sensing material allow, together with the absence of separation, or gap, the height of the sensor to be reduced, which in turn eases its integration into larger devices (e.g., CMOS circuitry, for integration into consumer electronics, smartphones or other mobile devices). Shallow sensors are furthermore preferred for batch manufacturing.

As present inventors have further realized, using a fluoropolymer layer, whose thickness is larger than 300nm, will substantially prevent reactive gases such as O₃ or NO₂ to reach the sensing material. Thus, in embodiments, the average thickness of the fluoropolymer layer 23 is between 300nm and 50µm, to block ingress of such reactive gases. This, in turn, allows the detection of small molecules (such as H₂, CO, CH₄, and C₂H₅OH) to be improved, even at low concentrations.

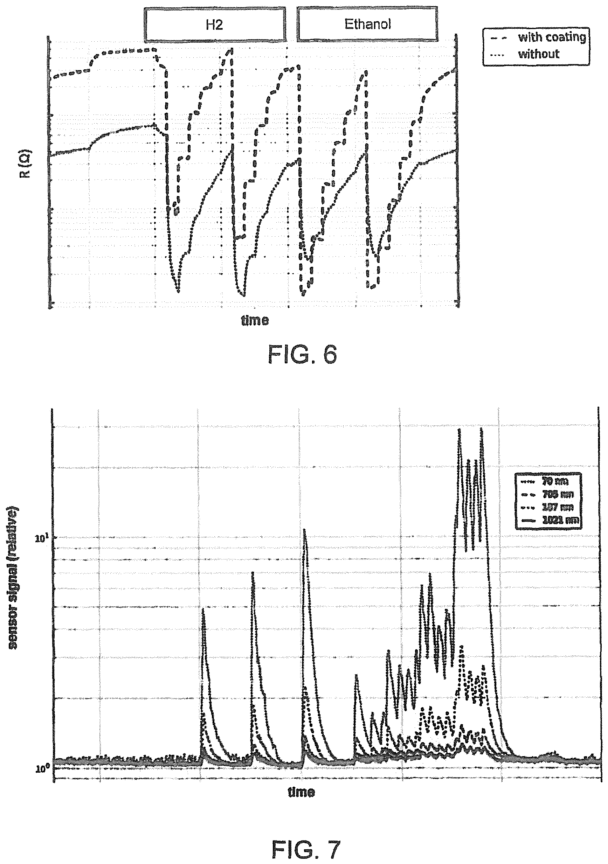

On the contrary, a thickness between 10nm and 300nm may be sought for the fluoropolymer layer 23, in order to specifically sense such reactive gases. Thus, in other embodiments, the average thickness of the fluoropolymer layer 23 is between 10nm and 300nm. For instance, FIG. 7 represents measured resistances of patches (SnO₂, doped with 1 Wt% Pd), coated with AF fluoropolymer layers of various thicknesses, while sensing ozone. The patches are subject to concentrations pulses of ozone. As illustrated in FIG. 7, the reaction of patches that have filter layers 23 thicker than 300nm to single pulses of gaseous ozone concentrations is just about discernable, contrary to patches having thinner fluoropolymer layers (less than 300nm thick), as seen between 20:00:00 and 00:00:00 timestamps. Thicker filters further make it difficult to sense gradually increasing concentrations of ozone (timestamps between 00:00:00 and 06:00:00 timestamps), whereas more discernable step-wise signals are observed with thinner filter layers 23 (i.e., of 70 and 187nm). Additional experiments, performed with other materials' compositions and dimensions, show that a thickness of 300nm provides, in general, a limit under which reactive gases (e.g., O₃, NO₂) can faithfully be sensed.

For instance, of particular advantage is to use a fluoropolymer layer 23, whose average thickness is between 50nm and 250nm, and a MOX material 21 that comprises SnO₂, doped with 0.01 - 1.0 Wt% platinum, which yields particularly good results for sensing ozone. The above ranges of dopants (in Wt%) denote ranges of mass percentage (also called weight percentage) of the dopants. For example, the range 0.01 - 1.0 Wt% is a range for the ratio of the Pt dopant to the mass of the total mixture (doped SnO₂), multiplied by 100 and subject to 0.005 accuracy. More generally, platinum and/or palladium may be used a dopant.

As evoked earlier, the gas-permeable filter may, in embodiments, comprise an interlayer 22, in addition to the fluoropolymer layer 23. In this case, the remaining part S2 of the external surface of the patch 21 is coated by the interlayer 22, which itself is coated by the layer 23 of fluoropolymer. The interlayer 22 is typically thin, e.g. between 300nm and 50µm. It preferably comprises a chemically inert material. In variants, it may also include a catalytically inactive layer, or, at least, a layer that is catalytically less active than the fluoropolymer 23. Catalytic activity can for instance be measured by thermogravimetric analysis. In other variants, it may comprise a polymer (e.g., PTFE). In practice, one shall preferably use materials such as SiO₂, MnO₂, Al₂O₃ or PTFE.

Referring now to FIG. 2, the present sensor can also be embodied as a multipixel-like gas sensor 1a. Namely, the sensor 1a now comprises a set of coated patches 2a - 2c, wherein each of the patches comprises a MOX material (not visible in FIG. 2), arranged on or partly housed in the support structure 6a, or on substructures 27, 29 thereof, and coated by a fluoropolymer layer 23. As illustrated in FIG. 2, the patches may be arranged on distinct substructures 27, 29 of the support structure 6a. In variants, the patches may be arranged on a same upper surface of the support structure (as if several patches were arranged in parallel on the support structure of FIG. 1).

In addition, the MOX material of each of the patches 2a - 2c is in electrical communication with a subset of electrodes, which are not visible either in FIG. 2. The electrodes may for instance be electrically exposed to the patches 2a - 2c thanks to suitably placed windows in a topmost dielectric layer (below the patches 2a - 2c). Furthermore, one or more heaters (not visible in FIG. 2) are in thermal communication with the patches of sensing material. The heaters may for example be formed out of a metal layer underneath the electrodes.

Consistently with the sensor of FIG. 1, one or more gas-permeable filters 22, 23 coats the MOX materials of the patches 2a - 2c, wherein the filters 22, 23 comprise, each, a fluoropolymer 23. The configuration of each patch 2a - 2c is otherwise similar to the patch 2 of FIG. 1. That is, for each patch 2a - 2c, a first part S1 of the external surface of its MOX material covers or coats a part of a support structure 6, 6a, 27, 29 (e.g., of substructures thereof), whereas a remaining part S2 of the external surface is coated by a gas-permeable filter 22, 23.

This way, distinct, coated patches 2a - 2c of sensing material are obtained. While similar patches may be used in parallel, e.g., for the sake of ascertaining or refining sensed values, the patches preferably differ, functionally speaking. They may notably differ: (i) in terms of dimensions and/or compositions of their respective gas-permeable filter 22, 23; and/or (ii) in terms of dimensions and/or compositions of their respective MOX materials. In addition, or in variants, the patches may functionally differ owing to the configurations of the electrodes, which may differ, from one patch to the other. Incidentally, the multipixel-like gas sensor may further include uncoated patches, in addition to the coated patches 2a - 2c.

The purpose is to be able to concomitantly and selectively sense various types of gas molecules. To that aim, and it can be realized, one may indeed vary the electrode configurations, the MOX materials and/or the filter materials, from one patch 2a - 2c to the other.

Most simple, however, is to solely vary the filters. Namely, the gas sensor 1a may comprise several, distinct gas-permeable filters 22, 23, e.g., each coating a top surface of the MOX materials of the patches. This way, fully distinct, coated patches 2a - 2c of sensing material are obtained, whose respective gas-permeable filters 22, 23 differ in terms of dimension and/or composition. Thus, the fabrication of the electrodes and heaters is unaffected.

Various configurations may be contemplated for the patches.
- For instance, one or more support structures may be relied on.
   ∘ The patches may for example be arranged across distinct substructures 27, 29, e.g., assuming bridge configurations, as discussed above in respect of FIG. 2, see also section 2.2 below.
   ∘ In variants, the heaters, electrodes and patches may be arranged on a same surface of the support structure, i.e., forming a common hotplate for all patches.
- Moreover, and as assumed in FIG. 2, distinct filter layer portions may cover respective MOX materials. One or each of the filter layer portions and the MOX materials may differ in terms of dimensions and/or compositions.
- In variants, a same filter layer may cover two or more (distinct) patches of MOX materials, to form partly distinct coated patches. Such coated patches may still differ in terms of dimensions and/or compositions of their respective MOX. Their function may further be differentiated, thanks to different electrodes configurations.
- Some of the patches may for instance include an interlayer 22, while other do not, etc.

Thus, several configurations can be contemplated, which enable selectivity of the sensed molecules.

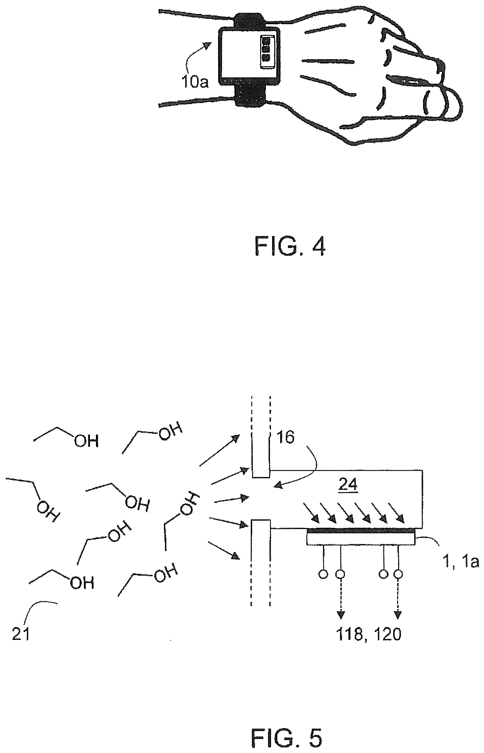

The present invention may further be embodied as an apparatus, e.g., comprising one or more of: a home automation device, a consumer electronics device, a mobile electronic device, in particular a mobile phone 10 (see FIG. 3), a tablet computer or a (smart)watch 10a (FIG. 4), comprising a resistive MOX gas sensor 1, 1a according to any of the embodiments discussed above. Exemplary embodiments directed to mobile devices are discussed in more detail in sect. 2.3.

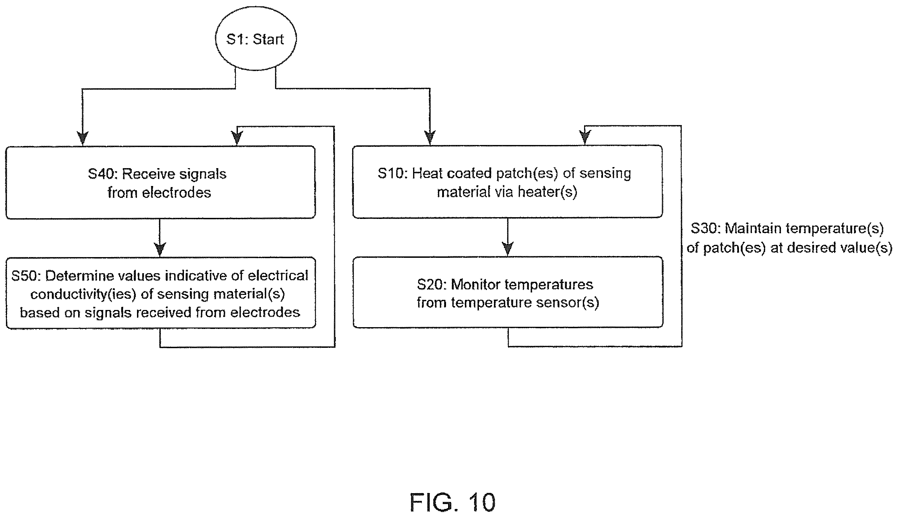

Next, according to another aspect, the invention can further be embodied as a method of operating a resistive MOX gas sensor 1, 1a (according to embodiments). Main aspects of such a method have already been described earlier and are here briefly discussed in reference to FIG. 10. Basically, in this method, a coated patch 2, 2a - 2c of sensing material is heated to an operating temperature, step S10, FIG. 10. Meanwhile, values indicative of an electrical conductivity of the sensing material 21 of the patch are determined (step S50), e.g., thanks to an evaluation unit 4, 4a, 118, 120), and based on signals received S40 from the electrodes. Steps S40, S50 may be continuously (i.e., repeatedly) performed, while heating the coated patch.

As discussed earlier, the patch 21 is preferably heated to a temperature that is between 100C and 300C. In particular, one may, while heating the coated patch, want to maintain a temperature of the coated patch at a desired value, e.g., below a glass transition temperature of the fluoropolymer. This can notably be achieved thanks to a feedback loop mechanism as described earlier. This, of course, does not preclude the possibility to occasionally heat the sensor for shorter periods to a temperature above the glass temperature of the fluoropolymer, e.g., to clean the sensor. Furthermore, intermittent heating is possible, as noted earlier.

Finally, if a "multipixel" gas sensor 1a is used, values indicative of the electrical conductivities of the sensing materials of the patches may be determined based on signals received S40 that pertain to distinct types of molecules. The latter are filtered by one or more gas-permeable filters 22, 23 and sensed via distinct, coated patches 2a - 2c of sensing material, as explained earlier in reference to FIG. 2.

The present approach makes it possible to clearly distinguish concentrations of gases (allowed by the filters 22, 23) to which the sensors are exposed. For example, FIG. 6 represents the resistances of different patches of sensing materials (which may be coated with a fluoropolymer, or not), after exposure to siloxane, and while sensing different types of gas molecules (i.e., dihydrogen and ethanol). Each curve denotes a measurement performed at 200C. The patches have been exposed to siloxane, prior to the measurements. It is known that siloxane, or more generally volatile Si compounds, may damage MOX sensors, whereby the response time of the sensors is usually increased and the sensor signal is reduced. This, however, can be prevented or at least mitigated by coating the MOX materials with a fluoropolymer, as illustrated in FIG. 6. As seen in FIG. 6, the sensor signals obtained from the coated patches remain substantially unaffected and, this, even after a few days of exposure to siloxane. In particular, the dashed curve (corresponding to the MOX material coated with fluoropolymer) shows a well-defined, stepwise structure, reflecting the gradual increase of concentration of the analyte (H₂, then Ethanol), while response times are not visibly impacted. The non-coated sensor, however, shows substantially reduced sensor signals after exposition to siloxane. In addition, the gradual increase of concentration of the analyte is not faithfully reflected. Thus, as one understands from FIG. 6, the performance and longevity of the sensor can both be improved, using a fluoropolymer that directly coats the MOX material.

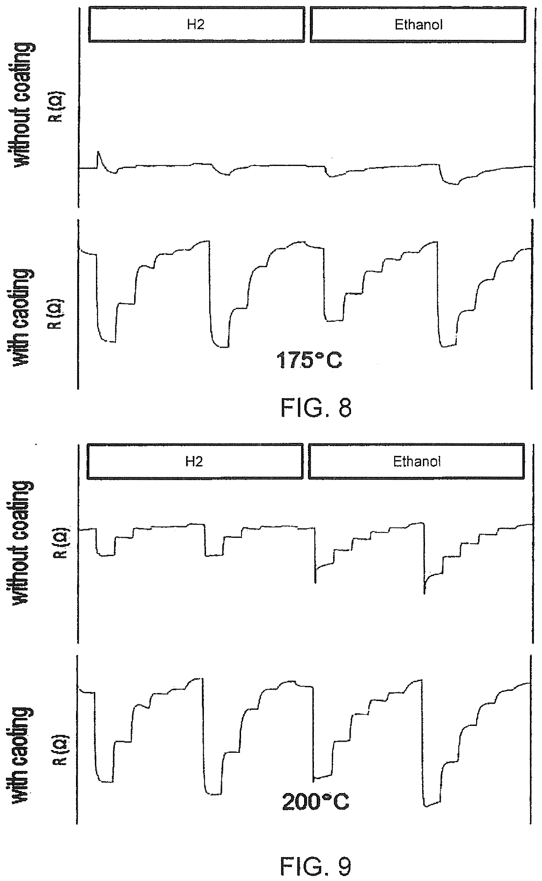

FIGS. 8 and 9 illustrate the influence of temperature on the measured resistances of coated and uncoated MOX patches when sensing different types of gas molecules (i.e., dihydrogen and ethanol). In each case, the signal obtained for uncoated patches has a smaller magnitude than the signal measured for patches coated with a fluoropolymer (Teflon AF was used in each case). While the signal obtained at 200C with the uncoated patch shows a stepwise structure (reflecting again a gradual increase of concentration of the analyte, the magnitude of the signal obtained with the uncoated patch collapses at lower temperature (175C, FIG. 8), unlike the coated patch's (FIG. 9). In fact, meaningful signals can still be measured at temperatures down to 100C with coated patches. The response times of the MOX sensors are not measurably impacted by the fluoropolymer coating.

The above embodiments have been succinctly described in reference to the accompanying drawings and may accommodate a number of variants. Several combinations of the above features may be contemplated. Examples are given in the next section.

### 2. Specific embodiments/Technical implementation details

### 2.1 Sensors in membrane configuration

Present gas sensors may be designed so as to have low thermal losses through the membrane. To that aim, the sensor may comprise a semiconductor substrate (such as a silicon substrate, forming a substantial part of the support structure 6) having a top and a bottom surface and an opening 12 extending between the top and bottom surfaces, as seen in FIG. 1. A batch of material layers 3, 5, e.g. comprising structured dielectric and metallic or semi-conducting layers, may be arranged on the top surface of the substrate, e.g. in order to form conducting leads and other electrical and electronic components of the device. Some of the material layers extend over the opening 12 of the semiconductor substrate, thereby forming a membrane. Further, a heater 5 is arranged on or in the membrane. Patches of sensing material can be patterned on an exposed surface of the membrane, on the side opposite the heater, with respect to this exposed surface

In addition, the device comprises a recess continuing the opening in the substrate and extending from below into the batch of material layers at the location of the membrane, thereby further reducing the thickness of the membrane and therefore thermal losses through the membrane.

A device of this type can be manufactured by providing a semiconductor substrate having a top and a bottom surface. The above-mentioned batch of material layers is applied to the top surface, and a heater is formed in the batch of material layers by suitable structuring techniques. Further, an opening is etched through the substrate, thereby forming a membrane formed by the material layers at the location of the heater. In addition, a recess is etched into the bottom side of the batch of material layers, namely by applying an etching agent through the opening in the substrate. In this manner, the thickness of the membrane can be further reduced.

This design is particularly advantageous when combined with modern CMOS processes, where typically a very large batch of material layers is applied to the semiconductor substrate. This batch can have a thickness of 10 µm or more, even at locations where the metal layers are removed. By forming said recess in the membrane, the thickness of the membrane can be optimized.

### 2.2 Sensors in bridge configuration

Present gas sensors may otherwise have a bridge configuration, whereby a high sensitivity can be obtained at low operating power. Such a gas sensor 1a comprises:
- A substrate: This substrate is, e.g., a silicon substrate forming a substantial part of the support structure 6a, see FIG. 2, and provides a mechanical frame for the sensor. It can optionally comprise circuitry 4a integrated thereon, in particular CMOS circuitry, and more particularly CMOS circuitry adapted to control a heater and to read out the gas sensor, based on signals received from the electrodes.
- A recess or opening 28 arranged in said substrate: This recess or opening is located under a thin structure forming the hotplate 26, thermally separating the hotplate from the substrate.
- A bridge 29 extending over said recess or opening and being anchored in said substrate: This bridge forms a thin structure with the hotplate, i.e., the bridge comprises the thin structure with the hotplate. By spanning the recess or opening by means of a bridge and not a continuous thin film cover, the thermal conductance between the hotplate and the substrate is reduced, thus allowing to achieve high temperatures at low operating power. Further, the thermal mass is reduced, which allows to vary the temperature of the hotplate quickly. The bridge comprises at least a first and a second metal layer separated by at least one dielectric layer. The dielectric layer is advantageously of at least one material selected from the group consisting of silicon oxide, silicon nitride, Al₂O₃ and Ta₂O₅. The bridge may for instance be anchored at opposite sides of the recess.
- A patch 2a - 2c of sensing material (MOX) arranged on said hotplate, wherein each MOX material is coated by a respective fluoropolymer layer portion 23, as assumed in FIG. 2. That is, distinct layer portions 23 coats each of the MOXs, to form fully distinct patches 2a - 2c.
- A heater (not visible in FIG. 2) located in said hotplate, to heat the hotplate to an operating temperature of the sensing material. It is formed by the first metal layer of the bridge.
- Electrodes (not visible in FIG. 2) located in said hotplate, for measuring an electrical property of said patch of sensing material, namely an electrical property depending on at least one gas analyte to be detected. The electrodes are formed at least in part by the second metal layer of the bridge. Advantageously, all said electrodes are formed by the second metal layer.
- A temperature sensor (not visible in FIG. 2) arranged in the hotplate. This temperature sensor is adapted to measure the temperature of the hotplate, and its signal can be used for refining the measurement and/or for controlling the hotplate temperature. In variants, the temperature sensor functionality may be provided by the heater, as noted earlier.

For example, FIG. 2 shows a gas sensor 1a having a support structure 6a that comprises a substrate, in particular a substrate of silicon, which confers its mechanical properties. The substrate has an opening or recess 28 arranged therein. Bridges 29 span this opening or recess. Furthermore, the substrate carries an integrated CMOS circuitry 4a, e.g., including circuitry for driving heaters and processing signals from the electrodes and temperature sensors. Advantageously, the processing circuitry 4a is integrated in CMOS technology since the whole device described in this section is compatible with current CMOS manufacturing processes. Having the CMOS circuitry on-board of the structure 6a allows to reduce the number of bonds to the substrate and to increase signal-to-noise ratios.

Each bridge 29 comprises a central region 25 forming a hotplate 26 and two arms 27 extending between the central region 26 and the substrate, thereby suspending the hotplate 26 over the recess or opening 28.

As can be seen in FIG. 2, there are advantageously exactly two arms 27 for each bridge 29, with the arms extending collinear to each other and with the central region 25 being arranged between them. The central regions 25 are not otherwise stabilized and therefore can have a tendency to tilt about the longitudinal axis of the arms 27. Thus, in variants, the central region 25 can also be suspended by more than two arms, thereby more securely preventing a tilting of the central region 25 in respect to the substrate, at the expense of increased thermal conduction between the central region 25 and the substrate.

A patch 2a - 2c of sensing material is arranged on each hotplate 26. The MOX sensing material changes at least one electrical property (in particular the real or imaginary part of its electrical impedance) as a function of the composition of the gas reaching it, if allowed by the fluoropolymer filter on top. The change of the property can be measured in order to obtain information on said composition.

### 2.3 Implementation in a mobile device

A gas sensor according to embodiments may be integrated in consumer goods such as a cloth, fabric, a handbag or a mobile device 10, 10a (FIGS. 3, 4). In the following, the gas sensor is assumed to be part of a hardware component integrated in a mobile device 10, 10a. The latter can notably be a smart companion device, a mobile phone 10 (as in FIG. 3) or a smartwatch 10a (FIG. 4).

In FIG. 3A, the housing of the mobile phone includes a front side with a screen 11 and other interface elements like buttons 12 for the user to interact with the phone 10, Also shown on the front side is an opening 13 for a loud-speaker. Further openings 14, 15 are located at a lower sidewall of the housing. It is known to mount components like microphones and loudspeakers behind such openings. Another opening 16 is located at the lower sidewall. As shown in FIG. 3B the opening 16 leads to a tubular duct passing through the interior of the housing.

As better seen in FIG. 5, the gas sensor 1 is preferably mounted along the duct such that a sensing side of the sensor is exposed to air 24 in a cavity formed inside the duct, which communicates with air outside the opening 16, so as for the gas sensor to be able to sense gaseous molecules diffusing from outside the opening 16 and propagating inside the duct. The actual size and shape of the duct may depend on the volume available therefor in the mobile device and the nature of the sensor 1. However, given the physical constraints of portable mobile devices, the diameter of the opening is typically less than 2 or even 1mm.

The gas sensor 1 may be embodied as any suitable gas sensor, e.g., of the chemiresistor type, having a membrane or bridge configuration. Any type of interactions between the sensitive element (the MOX layer) and the analyte may be involved, including chemical (e.g., covalent interactions), hydrogen bonds, van der Waals, etc.

As the dimensions of the opening and the inner volume of the duct may impact the amount of gaseous molecules reaching the sensor 1, the evaluation unit 4, 118, 120 may be equipped with algorithms that, or otherwise be configured to, compensate for this effect.

The sensor may be mounted on a PCB (not shown), further carrying other components, such as a chip (not shown). This chip may furthermore be operatively connected to one or more processors 120 and/or a sensor hub 118 of the mobile device 10, so as to carry out part or all of steps S10 - S40 (FIG. 10) and thereby off-load processing from the processors 120 and/or the sensor hub 118. In advantageous variants, the functions of the evaluation unit and temperature controller may be implemented directly at the sensor 1, as a CMOS circuitry 4. In particular, the evaluation unit and the temperature controller may be implemented as one and a same logical module, e.g., which may be implemented by a chip primarily dedicated to the sole gas sensor, or directly by said CMOS circuitry 4, so as for it to perform part or all of steps S10 - S40 and thereby offload processing from this chip or the remote components 118, 120.

### 2.4 Computerized devices and computer program products

Computerized devices can be suitably designed for implementing embodiments of the present invention as described herein. In that respect, it can be appreciated that the methods described herein are essentially non-interactive, i.e., automated. The methods described herein can be implemented as a combination of software (or, more generally, programmable instructions, in particular where programmable components 118, 120 are relied on, and/or logical operations) and hardware, or solely in hardware. In exemplary embodiments, the methods described herein use software or, more generally, and executable program or programmable instructions that are executed by suitable digital processing devices.

For instance, a typical electronic device 10 may include a processor 120 (FIG. 3B) and a memory coupled to a memory controller. The processor 120 is a hardware device for executing software, as, e.g., loaded in a main memory of the device. The processor 120 can be any custom made or commercially available processor. The above CMOS circuitry, chip and sensor hub may also be configured to execute computerized instructions or and/or logical operations (which may at least partly be hardcoded).

The memory typically includes a combination of volatile memory elements (e.g., random access memory) and nonvolatile memory elements, e.g., solid-state memory. The software in memory may include one or more separate programs, each of which comprises executable instructions for implementing logical functions. The software in the memory includes methods described herein in accordance with exemplary embodiments and, if necessary, a suitable operating system (OS). The OS essentially controls the execution of other computer (application) programs and provides scheduling, input-output control, file and data management, memory management, and communication control and related services. It may further control the distribution of tasks to be performed by units 118 and 120, as well as the CMOS circuitry 4 or chip dedicated to the gas sensor 1, as and if required by these components.

The methods described herein may typically use executable programs, scripts, or, more generally, any form of executable instructions.

When the device 10 is in operation, one or more of the processing elements 118, 120 are configured to execute software stored within the memory of the device 10 (possibly separate memory elements can be dedicated to each processing element 118, 120), to communicate data to and from the memory, and to generally control operations pursuant to software instructions. The methods described herein and the OS, are read by one or more of the processing elements, typically buffered therein, and then executed.

The device 10 can further include a display controller coupled to a display 11. In exemplary embodiments, the device 10 can further include a network interface or transceiver for coupling to a network.

Aspects of the present invention are described herein notably with reference to a flowchart (FIG. 10). It will be understood that at least some of the blocks, arrows or combinations thereof, denoting steps, can be implemented by computer readable program instructions or otherwise by logical operations.

Note that each block in the flowchart may represent a module, or a portion of instructions, which comprises executable instructions for implementing the functions or acts specified therein. In variants, the functions or acts mentioned in the blocks may occur out of the order specified in the figures. For example, two blocks shown in succession may actually be executed in parallel, concurrently or still in a reverse order, depending on the functions involved and the algorithm optimization retained. Each block and combinations thereof can be adequately distributed among special purpose hardware components, such as hardware accelerators.

While the present invention has been described with reference to a limited number of embodiments, variants and the accompanying drawings, it will be understood by those skilled in the art that various changes may be made and equivalents may be substituted without departing from the scope of the present invention. In particular, a feature (device-like or method-like) recited in a given embodiment, variant or shown in a drawing may be combined with or replace another feature in another embodiment, variant or drawing, without departing from the scope of the present invention. Various combinations of the features described in respect of any of the above embodiments or variants may accordingly be contemplated, that remain within the scope of the appended claims. In addition, many minor modifications may be made to adapt a particular situation or material to the teachings of the present invention without departing from its scope. Therefore, it is intended that the present invention not be limited to the particular embodiments disclosed, but that the present invention will include all embodiments falling within the scope of the appended claims. In addition, many other variants than explicitly touched above can be contemplated. For example, other MOX materials than those explicitly mentioned may be contemplated.

## Claims

1. A resistive metal oxide gas sensor (1, 1a), comprising
a support structure (6, 6a, 27, 29);
a patch (21) of sensing material, comprising a metal oxide material (21), the patch (21) arranged on the support structure (6, 6a, 27, 29) or partly housed therein;
electrodes (3) in electrical communication with the patch (21);
a heater (5), in thermal communication with the patch (21); and
a selective gas-permeable filter (22, 23),
wherein:
the selective gas-permeable filter (22, 23) comprises a fluoropolymer (23);
a first part (S1) of an external surface of the patch (21) covers a part of the support structure (6, 6a, 27, 29); and
a remaining part (S2) of said external surface is coated by the selective gas-permeable filter (22, 23), so as to form a coated patch (2, 2a - 2c) of sensing material.

2. The resistive metal oxide gas sensor (1,1a) according to claim **1,** further comprising
a temperature controller (4, 4a, 118, 120),
wherein the temperature controller (4, 4a, 118, 120)
is an electronic circuit or processing unit connected to the heater (5), and
is programmed, designed, adapted or configured to prevent the heater (5) to heat the coated patch (2, 2a - 2c) to a temperature exceeding 300C.

3. The resistive metal oxide gas sensor (1,1a) according to claim **1,** further comprising:
a temperature sensor (5) arranged in the resistive metal oxide gas sensor (1,1a), for estimating a temperature of the coated patch (2, 2a - 2c), wherein the temperature sensor preferably forms a part of the heater (5); and
a temperature controller (4, 4a, 118, 120) connected to the heater (5) and, if necessary, to the temperature sensor (5),
wherein the temperature controller (4, 4a, 118, 120)
is an electronic circuit or processing unit, and
is programmed, designed, adapted or configured to form, together with said heater (5) and, if necessary, said temperature sensor, a feedback loop, so as to maintain, in operation, a temperature of said coated patch (2, 2a - 2c) at a substantially constant value, below a glass transition temperature of the fluoropolymer.

4. The resistive metal oxide gas sensor (1,1a) according to claim **3**, wherein
said feedback loop is so as to maintain, in operation, a temperature of said coated patch (2, 2a - 2c) at a substantially constant value between 100C and 240C, and preferably between 150C and 200C.

5. The resistive metal oxide gas sensor (1,1a) according to any one of claims **1** to **4,** wherein
said fluoropolymer (23) comprises Teflon, preferably amorphous fluoroplastic Teflon, and more preferably Teflon AF 1600 or Teflon AF 2400.

6. The resistive metal oxide gas sensor (1, 1a) according to claim **1** or **5,** wherein:
said selective gas-permeable filter (22, 23) comprises one or more layers of materials, the one or more layers including a fluoropolymer layer (23), the latter comprising said fluoropolymer; and
an average thickness of the fluoropolymer layer (23) is between 10nm and 50µm, and preferably between 10nm and 2µm.

7. The resistive metal oxide gas sensor (1, 1a) according to claim **6,** wherein
an average thickness of the fluoropolymer layer (23) is between 300nm and 50µm.

8. The resistive metal oxide gas sensor (1, 1a) according to claim **6,** wherein
an average thickness of the fluoropolymer layer (23) is between 10nm and 300nm.

9. The resistive metal oxide gas sensor (1, 1a) according to claim **8,** wherein
the average thickness of the fluoropolymer layer (23) is between 50nm and 250nm, and
said metal oxide material (21) comprises SnO₂, doped with 0.01 - 1.0 Wt% platinum and/or palladium.

10. The resistive metal oxide gas sensor (1, 1a) according to any one of claims **1** to **9,** further comprising an evaluation unit (4, 4a, 118, 120),
wherein,
the evaluation unit (4, 4a, 118, 120) is an electronic circuit or processing unit connected to said electrodes (3) to receive signals therefrom, and is programmed, designed, adapted or configured to determine values indicative of an electrical conductivity of the metal oxide material (21) based on signals received from the electrodes.

11. The resistive metal oxide gas sensor (1, 1a) according to any one of claims **1** to **10,** wherein
an average thickness of the sensing material of the patch (21) is between 0.1µm and 50µm, and preferably between 0.5µm and 5µm.

12. The resistive metal oxide gas sensor (1, 1a) according to any one of claims **1** to **11,** wherein
said selective gas-permeable filter comprises a layer (23) of said fluoropolymer and an interlayer (22), wherein said remaining part (S2) of the external surface of the patch (21) is coated by the interlayer (22), the latter coated by the layer (23) of fluoropolymer.

13. The resistive metal oxide gas sensor (1, 1a) according to any one of claims **1** to **12,** wherein the first part (S1) of the external surface coats said part of the support structure (6, 6a, 27, 29).

14. The resistive metal oxide gas sensor (1, 1a) according to claim **1,**
wherein the sensor comprises:
one or more support structures (1, 27, 29), including said support structure;
a set of patches (21) of sensing material, including said patch (21), wherein each of the patches comprises a metal oxide material (21) and is arranged on or partly housed in one of said one or more support structures;
a set of electrodes (3), including said electrodes, whereby each of the patches is in electrical communication with a subset of the electrodes (3) of said set;
one or more heaters (5), including said heater (5), wherein the one or more heaters are in thermal communication with the patches (21) of sensing material; and
one or more selective gas-permeable filters (22, 23), including said selective gas-permeable filter,
wherein:
the one or more selective gas-permeable filters (22, 23) comprise, each, a fluoropolymer (23);
a first part (S1) of an external surface of each of the patches covers a part of a respective one of said one or more support structures (1, 27, 29);
a remaining part (S2) of the external surface of each of the patches (21) is coated by one of the one or more selective gas-permeable filters (22, 23), so as to form distinct, coated patches (2a - 2c) of sensing material; and
said distinct, coated patches differ in terms of dimensions and/or compositions of respective selective gas-permeable filters (22, 23) and/or respective metal oxide materials, or said distinct, coated patches are in electrical communication with respective subsets of electrodes that have different configurations.

15. A resistive metal oxide gas sensor (1, 1a) according to claim **14,** comprising several, distinct selective gas-permeable filters (22, 23), wherein
the remaining part (S2) of the external surface of two or more of the patches is coated by a respective one of the several, distinct selective gas-permeable filters (22, 23), to form distinct, coated patches (2, 2a - 2c) of sensing material, whose respective selective gas-permeable filters (22, 23) differ in terms of dimension and/or composition.

16. An electronic device, such as a home automation device, a consumer electronics device, a mobile phone, a tablet computer or a watch, comprising the resistive metal oxide gas sensor (1, 1a) according to any one of claims **1** to **15.**

17. A method of operating a resistive metal oxide gas sensor (1, 1a) according to any one of claims **1** to **15,** the method comprising:
heating a coated patch (2, 2a - 2c) of sensing material; and
determining values indicative of an electrical conductivity of the sensing material (21) of the patch, based on signals received from the electrodes (3), while heating the coated patch.

18. The method according to claim **17,** wherein
the patch (21) is heated to a temperature that is between 100C and 300C.

19. The method according to claim **18,** wherein
heating the coated patch of sensing material further comprises maintaining a temperature of said coated patch at a desired value, below a glass transition temperature of the fluoropolymer.

20. A method according to any one of claims **17** to **19,** for operating a resistive metal oxide gas sensor (1, 1a) according to claim **14** or **15,** the method comprising
receiving (S40) signals pertaining to distinct types of molecules, as filtered by the one or more selective gas-permeable filters (22, 23) and sensed via the distinct, coated patches (2, 2a - 2c) of sensing material.

## Patentansprüche

1. Ein resistiver Metalloxid-Gassensor (1, 1a), umfassend
eine Trägerstruktur (6, 6a, 27, 29);
einen Fleck (21) aus Sensormaterial, umfassend ein Metalloxidmaterial (21), wobei der Fleck (21) auf der Trägerstruktur (6, 6a, 27, 29) angeordnet oder teilweise darin untergebracht ist;
Elektroden (3) in elektrischer Verbindung mit dem Fleck (21);
eine Heizvorrichtung (5), die in thermischer Verbindung mit dem Fleck (21) steht; und ein selektiver gasdurchlässiger Filter (22, 23),
wobei:
der selektive gasdurchlässige Filter (22, 23) ein Fluorpolymer (23) umfasst;
ein erster Teil (S1) einer äusseren Oberfläche des Flecks (21) einen Teil der Trägerstruktur (6, 6a, 27, 29) bedeckt; und
ein verbleibender Teil (S2) der äusseren Oberfläche durch den selektiven gasdurchlässigen Filter (22, 23) beschichtet ist, so dass ein beschichteter Fleck (2, 2a - 2c) aus Sensormaterial gebildet wird.

2. Der resistive Metalloxid-Gassensor (1, 1a) nach Anspruch **1,** weiter umfassend
einem Temperaturregler (4, 4a, 118, 120),
wobei der Temperaturregler (4, 4a, 118, 120)
eine elektronische Schaltung oder Verarbeitungseinheit ist, die mit der Heizvorrichtung (5) verbunden ist, und
programmiert, konstruiert, angepasst oder konfiguriert ist, um zu verhindern, dass die Heizvorrichtung (5) den beschichteten Fleck (2, 2a - 2c) auf eine Temperatur von mehr als 300C erhitzt.

3. Der resistive Metalloxid-Gassensor (1, 1a) nach Anspruch **1,** weiter umfassend:
einen Temperatursensor (5), der in dem resistiven Metalloxid-Gassensor (1, 1a) angeordnet ist, zum Abschätzen einer Temperatur des beschichteten Flecks (2, 2a - 2c), wobei der Temperatursensor vorzugsweise einen Teil der Heizvorrichtung (5) bildet; und
einen Temperaturregler (4, 4a, 118, 120), der mit der Heizvorrichtung (5) und, falls erforderlich, mit dem Temperatursensor (5) verbunden ist,
wobei der Temperaturregler (4, 4a, 118, 120)
eine elektronische Schaltung oder Verarbeitungseinheit ist, und
programmiert, konstruiert, angepasst oder konfiguriert ist, um zusammen mit der Heizvorrichtung (5) und, falls erforderlich, dem Temperatursensor eine Rückkopplungsschleife zu bilden, so dass im Betrieb eine Temperatur des beschichteten Flecks (2, 2a - 2c) auf einem im Wesentlichen konstanten Wert unterhalb einer Glasübergangstemperatur des Fluorpolymers gehalten wird.

4. Der resistive Metalloxid-Gassensor (1, 1a) nach Anspruch **3,** wobei
die Rückkopplungsschleife so ausgelegt ist, dass sie im Betrieb eine Temperatur des beschichteten Flecks (2, 2a - 2c) auf einem im Wesentlichen konstanten Wert zwischen 100C und 240C, und vorzugsweise zwischen 150C und 200C, hält.

5. Der resistive Metalloxid-Gassensor (1, 1a) nach einem der Ansprüche**1** bis **4,** wobei
das Fluorpolymer (23) Teflon umfasst, vorzugsweise amorphen fluoroplastisches Teflon, und noch bevorzugter Teflon AF 1600 oder Teflon AF 2400.

6. Der resistive Metalloxid-Gassensor (1, 1a) nach Anspruch **1** oder**5**, wobei:
der selektive gasdurchlässige Filter (22, 23) eine oder mehrere Materialschichten umfasst, wobei die eine oder die mehreren Schichten eine Fluorpolymerschicht (23) enthalten, wobei letztere das Fluorpolymer umfasst; und
eine durchschnittliche Dicke der Fluorpolymerschicht (23) zwischen 10 nm und 50µm, und vorzugsweise zwischen 10 nm und 2µm liegt.

7. Der resistive Metalloxid-Gassensor (1, 1a) nach Anspruch **6,** wobei
eine durchschnittliche Dicke der Fluorpolymerschicht (23) zwischen 300 nm und 50µm liegt.

8. Der resistive Metalloxid-Gassensor (1, 1a) nach Anspruch **6,** wobei
die durchschnittliche Dicke der Fluorpolymerschicht (23) zwischen 10 nm und 300 nm liegt.

9. Der resistive Metalloxid-Gassensor (1, 1a) nach Anspruch **8,** wobei
die durchschnittliche Dicke der Fluorpolymerschicht (23) zwischen 50 nm und 250 nm liegt, und
das Metalloxidmaterial (21) SnO2 umfasst, das mit 0,01 - 1,0 Gew.-% Platin und/oder Palladium dotiert ist.

10. Der resistive Metalloxid-Gassensor (1, 1a) nach einem der Ansprüche **1** bis **9,** weiter umfassend eine Auswerteeinheit (4, 4a, 118, 120),
wobei,
die Auswerteeinheit (4, 4a, 118, 120) eine elektronische Schaltung oder Verarbeitungseinheit ist, die mit den Elektroden (3) verbunden ist, um Signale von diesen zu empfangen, und programmiert, entworfen, angepasst oder konfiguriert ist, um Werte zu bestimmen, die indikativ sind für eine elektrische Leitfähigkeit des Metalloxidmaterials (21) auf der Grundlage von von den Elektroden empfangenen Signalen.

11. Der resistive Metalloxid-Gassensor (1, 1a) nach einem der Ansprüche **1** bis **10,** wobei
eine durchschnittliche Dicke des Sensormaterials des Flecks (21) zwischen 0,1µm und 50µm liegt, und vorzugsweise zwischen 0,5µm und 5µm.

12. Der resistive Metalloxid-Gassensor (1, 1a) nach einem der Ansprüche **1** bis **11,** wobei
der selektive gasdurchlässige Filter eine Schicht (23) aus dem Fluorpolymer und eine Zwischenschicht (22) umfasst, wobei der verbleibende Teil (S2) der äusseren Oberfläche des Flecks (21) mit der Zwischenschicht (22) beschichtet ist, wobei letztere mit der Schicht (23) aus Fluorpolymer beschichtet ist.

13. Der resistive Metalloxid-Gassensor (1, 1a) nach einem der Ansprüche **1** bis **12,** wobei
der erste Teil (S1) der äusseren Oberfläche den Teil der Trägerstruktur (6, 6a, 27, 29) beschichtet.

14. Der resistive Metalloxid-Gassensor (1, 1a) nach Anspruch **1,**
wobei der Sensor umfasst:
eine oder mehrere Trägerstrukturen (1, 27, 29), einschliesslich der genannten Trägerstruktur;
einen Satz von Flecken (21) aus Sensormaterial, einschliesslich des Fleckes (21), wobei jeder der Flecken ein Metalloxidmaterial (21) umfasst und auf oder teilweise in einer der einen oder mehreren Trägerstrukturen angeordnet ist;
einen Satz von Elektroden (3), einschliesslich der genannten Elektroden, wobei jeder der Flecken in elektrischer Verbindung mit einer Teilmenge der Elektroden (3) des genannten Satzes steht;
eine oder mehrere Heizvorrichtungen (5), einschliesslich der genannten Heizvorrichtung (5), wobei die eine oder die mehreren Heizvorrichtungen in thermischer Verbindung mit den Flecken (21) des Sensormaterials stehen; und
einen oder mehrere selektive gasdurchlässige Filter (22, 23), einschliesslich des genannten selektiven gasdurchlässigen Filters,
wobei:
der eine oder die mehreren selektiven gasdurchlässigen Filter (22, 23) jeweils ein Fluorpolymer (23) umfassen;
ein erster Teil (S1) einer äussern Oberfläche jedes der Flecken einen Teil einer entsprechenden der genannten einen oder mehreren Trägerstrukturen (1, 27, 29) bedeckt;
ein verbleibender Teil (S2) der äusseren Oberfläche jedes der Flecken (21) durch einen der einen oder mehrere selektiven gasdurchlässige Filter (22, 23) beschichtet ist, um so verschiedene beschichtete Flecken (2a - 2c) aus Sensormaterial zu bilden; und
sich die verschiedenen, beschichteten Flecken in Bezug auf die Abmessungen und/oder die Zusammensetzungen der jeweiligen selektiven gasdurchlässigen Filter (22, 23) und/oder die jeweiligen Metalloxidmaterialien unterscheiden, oder die verschiedenen, beschichteten Flecken in elektrischer Verbindung mit jeweiligen Untergruppen von Elektroden stehen, die unterschiedliche Konfigurationen aufweisen.

15. Ein resistiver Metalloxid-Gassensor (1, 1a) nach Anspruch **14,** umfassend mehrere, unterschiedliche selektive gasdurchlässige Filter (22, 23), wobei
der verbleibende Teil (S2) der äusseren Oberfläche von zwei oder mehreren der Flecken durch einen jeweiligen der mehreren, unterschiedlichen, selektiven gasdurchlässigen Filter (22, 23) beschichtet wird, um unterschiedliche, beschichtete Flecken (2, 2a - 2c) aus Sensormaterial zu bilden, deren jeweilige selektive, gasdurchlässige Filter (22, 23) sich in Bezug auf die Dimension und/oder Zusammensetzung unterscheiden.

16. Ein elektronisches Gerät, wie zum Beispiel ein Hausautomatisierungsgerät, ein Gerät der Unterhaltungselektronik, ein Mobiltelefon, ein Tablet-Computer oder eine Uhr, mit einem resistiven Metalloxid-Gassensor (1, 1a) nach einem der Ansprüche **1** bis **15.**

17. Ein Verfahren zum Betreiben eines resistiven Metalloxid-Gassensors (1, 1a) nach einem der Ansprüche **1** bis **15,** wobei das Verfahren umfasst:
Erwärmung eines beschichteten Flecks (2, 2a - 2c) aus Sensormaterial; und
Bestimmen von Werten, die indikativ sind für eine elektrische Leitfähigkeit des Sensormaterials (21) des Flecks, auf der Grundlage von Signalen, die von den Elektroden (3) empfangen werden, während der beschichtete Fleck erhitzt wird.

18. Das Verfahren nach Anspruch **17,** wobei
wird der Fleck (21) auf eine Temperatur zwischen 100C und 300C erhitzt wird.

19. Das Verfahren nach Anspruch **18,** wobei
das Erwärmen des beschichteten Flecks aus Sensormaterial ferner das Beibehalten einer Temperatur des beschichteten Flecks auf einem gewünschten Wert unterhalb einer Glasübergangstemperatur des Fluorpolymers umfasst.

20. Ein Verfahren nach einem der Ansprüche **17** bis **19** zum Betrieb eines resistiven Metalloxid-Gassensors (1, 1a) nach Anspruch **14** oder1**5,** wobei das Verfahren umfasst
Empfangen (S40) von Signalen, die sich auf verschiedene Arten von Molekülen beziehen, wie sie von dem einem oder mehreren selektiven gasdurchlässigen Filtern (22, 23) gefiltert werden und mittels der verschiedenen, beschichteten Flecken (2, 2a - 2c) des Sensormaterials detektiert werden.

## Revendications

1. Un capteur de gaz à oxyde métallique résistif (1, 1a), comprenant
une structure de support (6, 6a, 27, 29) ;
une pastille (21) de matériau de détection, comprenant un matériau d'oxyde métallique (21), la pastille (21) disposée sur la structure de support (6, 6a, 27, 29) ou partiellement logée à l'intérieur de celle-ci ;
des électrodes (3) en communication électrique avec la pastille (21) ;
un élément chauffant (5), en communication thermique avec la pastille (21) ; et
un filtre sélectif perméable aux gaz (22, 23),
dans lequel :
le filtre sélectif perméable aux gaz (22, 23) comprend un fluoropolymère (23) ;
une première partie (S1) d'une surface externe de la pastille (21) recouvre une partie de la structure de support (6, 6a, 27, 29) ; et
une partie restante (S2) de ladite surface externe est recouverte par le filtre sélectif perméable aux gaz (22, 23), de manière à former une pastille (2, 2a - 2c) de matériau de détection recouverte.

2. Le capteur de gaz à oxyde métallique résistif (1, 1a) selon la revendication **1,** comprenant en outre
un régulateur de température (4, 4a, 118, 120),
dans lequel le régulateur de température (4, 4a, 118, 120)
est un circuit électronique ou une unité de traitement connecté(e) à l'élément chauffant (5), et
est programmé(e), conçu(e), adapté(e) ou configuré(e) pour empêcher l'élément chauffant (5) de chauffer la pastille recouverte (2, 2a - 2c) à une température excédant 300C.

3. Le capteur de gaz à oxyde métallique résistif (1, 1a) selon la revendication **1,** comprenant en outre :
un capteur de température (5) disposé dans le capteur de gaz d'oxyde métallique résistif (1, 1a), pour estimer une température de la pastille recouverte (2, 2a - 2c), dans lequel le capteur de température fait de préférence partie de l'élément chauffant (5) ; et
un régulateur de température (4, 4a, 118, 120) connecté à l'élément chauffant (5) et, si nécessaire, au capteur de température (5),
dans lequel le régulateur de température (4, 4a, 118, 120)
est un circuit électronique ou une unité de traitement, et
est programmé, conçu, adapté ou configuré pour former, avec ledit élément chauffant (5) et, si nécessaire, ledit capteur de température, une boucle de rétroaction, de manière à maintenir, en fonctionnement, une température de ladite pastille recouverte (2, 2a - 2c) à une valeur sensiblement constante, inférieure à une température de transition vitreuse du fluoropolymère.

4. Le capteur de gaz à oxyde métallique résistif (1, 1a) selon la revendication **3,** dans lequel
ladite boucle de rétroaction est de telle sorte à maintenir, en fonctionnement, une température de ladite pastille recouverte (2, 2a - 2c) à une valeur sensiblement constante entre 100C et 240C, et de préférence entre 150C et 200C.

5. Le capteur de gaz à oxyde métallique résistif (1, 1a) selon l'une quelconque des revendications **1** à **4,** dans lequel
ledit fluoropolymère (23) comprend du téflon, de préférence du téflon fluoroplastique amorphe, et plus préférablement du téflon AF 1600 ou du téflon AF 2400.

6. Le capteur de gaz à oxyde métallique résistif (1, 1a) selon la revendication Fehler! Verweisquelle konnte nicht gefunden werden. ou **5,** dans lequel :
ledit filtre sélectif perméable aux gaz (22, 23) comprend une ou plusieurs couches de matériaux, la ou les couches comprenant une couche de fluoropolymère (23), cette dernière comprenant ledit fluoropolymère; et
une épaisseur moyenne de la couche de fluoropolymère (23) est comprise entre 10 nm et 50 µm, et de préférence entre 10 nm et 2 µm.

7. Le capteur de gaz à oxyde métallique résistif (1, 1a) selon la revendication **6,** dans lequel :
une épaisseur moyenne de la couche de fluoropolymère (23) est comprise entre 300 nm et 50 µm.

8. Le capteur de gaz à oxyde métallique résistif (1, 1a) selon la revendication **6,** dans lequel
une épaisseur moyenne de la couche de fluoropolymère (23) est comprise entre 10 nm et 300 nm.

9. Le capteur de gaz à oxyde métallique résistif (1, 1a) selon la revendication **8,** dans lequel
l'épaisseur moyenne de la couche de fluoropolymère (23) est comprise entre 50 nm et 250 nm, et
ledit matériau d'oxyde métallique (21) comprend du SnO₂, dopé avec du platine et / ou du palladium à 0,01 à 1,0% en pourcentage massique.

10. Le capteur de gaz à oxyde métallique résistif (1, 1a) selon l'une quelconque des revendications **1** à **9,** comprenant en outre une unité d'évaluation (4, 4a, 118, 120),
dans lequel,
l'unité d'évaluation (4, 4a, 118, 120) est un circuit électronique ou une unité de traitement connecté(e) auxdites électrodes (3) pour en recevoir des signaux, et est programmée, conçue, adaptée ou configurée pour déterminer des valeurs indicatives d'une conductivité électrique du matériau d'oxyde métallique (21) sur la base des signaux reçus des électrodes.

11. Le capteur de gaz à oxyde métallique résistif (1, 1a) selon l'une quelconque des revendications **1** à **10,** dans lequel
une épaisseur moyenne du matériau de détection de la pastille (21) est comprise entre 0,1 µm et 50 µm, et de préférence entre 0,5 µm et 5 µm.

12. Le capteur de gaz à oxyde métallique résistif (1, 1a) selon l'une quelconque des revendications **1** à **11,** dans lequel
ledit filtre perméable aux gaz sélectif comprend une couche (23) dudit fluoropolymère et une couche intermédiaire (22), ladite partie restante (S2) de la surface externe de la pastille (21) étant recouverte par la couche intermédiaire (22), cette dernière étant recouverte par la couche (23) de fluoropolymère.

13. Le capteur de gaz à oxyde métallique résistif (1, 1a) selon l'une quelconque des revendications **1** à **12,** dans lequel
la première partie (S1) de la surface externe recouvre ladite partie de la structure de support (6, 6a, 27, 29).

14. Le capteur de gaz à oxyde métallique résistif (1, 1a) selon la revendication **1,**
dans lequel le capteur comprend :
une ou plusieurs structures de support (1, 27, 29), lesquelles comprennent ladite structure de support ;
un ensemble de pastilles (21) de matériau de détection, lequel ensemble comprend ladite pastille (21), dans lequel chacune des pastilles comprend un matériau d'oxyde métallique (21) et est disposée sur ou partiellement logée dans l'une desdites une ou plusieurs structures de support ;
un ensemble d'électrodes (3), comprenant lesdites électrodes, chacune des pastilles étant en communication électrique avec un sous-ensemble des électrodes (3) dudit ensemble d'électrodes ;
un ou plusieurs éléments chauffants (5), lesquels comprennent ledit élément chauffant (5), ledit un ou plusieurs éléments chauffants étant en communication thermique avec les pastilles (21) de matériau de détection ; et
un ou plusieurs filtres sélectifs perméables aux gaz (22, 23), comprenant ledit filtre sélectif perméable aux gaz,
dans lequel :
le ou les filtres sélectifs perméables aux gaz (22, 23) comprennent chacun un fluoropolymère (23) ;
une première partie (S1) d'une surface externe de chacune des pastilles recouvre une partie d'une structure respective desdites une ou plusieurs structures de support (1, 27, 29) ;
une partie restante (S2) de la surface externe de chacune des pastilles (21) est recouverte par l'un desdits un ou plusieurs filtres sélectifs perméables aux gaz (22, 23), de manière à former des pastilles distinctes (2a - 2c) de matériau de détection, recouvertes ; et
lesdites pastilles recouvertes distinctes diffèrent en termes de dimensions et / ou de compositions de filtres sélectifs perméables aux gaz respectifs (22, 23) et / ou de matériaux d'oxyde métallique respectifs, ou lesdites pastilles recouvertes distinctes sont en communication électrique avec des sous-ensembles respectifs d'électrodes qui ont des configurations différentes.

15. Un capteur résistif d'oxyde de métal (1, 1a) selon la revendication **14,** comprenant plusieurs filtres sélectifs perméables aux gaz (22, 23), dans lesquels
la partie restante (S2) de la surface externe de deux ou plusieurs des pastilles est recouverte par un filtre respectif des filtres sélectifs perméables aux gaz (22, 23), pour former des pastilles distinctes (2, 2a - 2c) de matériau de détection, recouvertes, dont les filtres sélectifs respectifs, perméables aux gaz (22, 23), diffèrent en termes de dimension et / ou de composition.

16. Un dispositif électronique, tel qu'un dispositif domotique, un dispositif électronique grand public, un téléphone portable, une tablette ou une montre, comprenant le capteur de gaz à oxyde métallique résistif (1, 1a) selon l'une quelconque des revendications **1** à **15.**

17. Un procédé de fonctionnement d'un capteur de gaz à oxyde métallique résistif (1, 1a) selon l'une quelconque des revendications **1** à **15,** le procédé comprenant :
chauffer une pastille (2, 2a - 2c) de matériau de détection recouverte ; et
déterminer des valeurs indicatives d'une conductivité électrique du matériau de détection (21) de la pastille, sur la base des signaux reçus des électrodes (3), tout en chauffant la pastille recouverte.

18. Le procédé selon la revendication **17,** dans lequel
La pastille (21) est chauffée à une température comprise entre 100C et 300C.

19. Le procédé selon la revendication **18,** dans lequel
chauffer la pastille de matériau de détection recouverte comprend en outre le maintien d'une température de ladite pastille recouverte à une valeur souhaitée, en dessous d'une température de transition vitreuse du fluoropolymère.

20. Un procédé selon l'une quelconque des revendications **17** à **19,** pour faire fonctionner un capteur de gaz à oxyde métallique résistif (1, 1a) selon la revendication **14** ou **15,** le procédé comprenant
recevoir (S40) des signaux se rapportant à des types de molécules distincts, filtrés par lesdits un ou plusieurs filtres sélectifs perméables aux gaz (22, 23) et détectés par les pastilles recouvertes distinctes (2, 2a - 2c) de matériau de détection.
